# EUROPÄISCHE PATENTSCHRIFT

(11) **EP 0 662 005 B1**
(45) Veröffentlichungstag und Bekanntmachung des Hinweises auf die Patenterteilung: **29.04.1998**
(21) Anmeldenummer: 93919293.6
(22) Anmeldetag: 08.09.1993
(51) Int. Cl.: A61K 49/00

(54) **MIKROPARTIKELPRÄPARATIONEN AUS BIOLOGISCH ABBAUBAREN MISCHPOLYMEREN**
MICROPARTICLE PREPARATIONS MADE FROM BIODEGRADABLE COPOLYMERS
PREPARATIONS DE MICROPARTICULES A BASE DE COPOLYMERES BIODEGRADABLES

(30) Priorität: 26.09.1992 DE 4232755
(43) Veröffentlichungstag der Anmeldung: 12.07.1995
(73) Patentinhaber: SCHERING AKTIENGESELLSCHAFT, 13342 Berlin (DE)
(72) Erfinder: FRITZSCH, Thomas, Dr., D-12169 Berlin (DE); HELDMANN, Dieter, Dr., D-10555 Berlin (DE); WEITSCHIES, Werner, Dr., D-10961 Berlin (DE)
(86) Internationale Anmeldenummer: EP9302422
(87) Internationale Veröffentlichungsnummer: WO9407539

(56) Entgegenhaltungen:
- EP-A- 0 327 490
- EP-A- 0 441 468
- EP-A- 0 458 745
- WO-A-92/17213
- BRITISH MEDICAL JOURNAL Bd. 1, Nr. 5952 , Februar 1975 , (LONDON) Seiten 247 - 249 J.P. BOLTON ET AL. 'INCIDENCE OF EARLY POST-OPERATIVE ILIOFEMORAL THROMBOSIS'

## Beschreibung

Die Erfindung betrifft den in den Patentansprüchen gekennzeichneten Gegenstand, d.h. aus Biopolymeren, zur Polymerisation befähigten Monomeren, Wirkstoffen und/oder diagnostisch nachweisbaren Bestandteilen hergestellte Mikropartikel, ein Verfahren zu deren Herstellung sowie deren Verwendung in Diagnostik und Therapie, insbesondere als Kontrastmittel in der Ultraschalldiagnostik. Die erfindungsgemäß Mikropartikel sind Mikrokapseln.

Es ist bekannt, daß Partikel, deren Durchmesser kleiner oder im Bereich der Größe roter Blutzellen ist, nach Injektion in die Blutstrombahn innerhalb des Blutgefäßsystems zirkulieren können. Pharmazeutische Präparationen derartiger Mikropartikel eignen sich deshalb als in das Blutgefäßsystem injizierbare Trägersysteme für Wirkstoffe oder Diagnostika in der Medizin. Als Trägermaterialien kommen prinzipiell alle biologisch abbaubaren, verträglichen und nicht wasserlöslichen Stoffe in Betracht. Beschrieben sind bisher vor allem Fette, Wachse, Lipide (z.B. Sojalecithin), denaturierte Biopolymere (z.B. Albumin, Gelatine) und synthetische biologisch abbaubare Polymere (z. B. Polymilchsäure, Polyhydroxybuttersäure, Polyalkylcyanoacrylate, Poly-L-Lysin).

Die in der Blutstrombahn zirkulierenden Mikropartikel werden in Abhängigkeit von ihren physikalischen und chemischen Eigenschaften unterschiedlich schnell und in unterschiedlicher Anzahl durch die Zellen des monozytären phagozytierenden Systems (MPS) erkannt und aufgenommen (vorwiegend in Leber, Lunge und Milz). Als wesentliche Faktoren, die die Kinetik der Aufnahme der Mikropartikel durch die Zellen des MPS bestimmen, werden die Partikelladung, die Partikelgröße, die Eigenschaften (Molekulargewicht, Amphiphilie) an der Partikeloberfläche adsorbierter Substanzen, sowie die Affinität der Partikeloberfläche für Blutkomponenten wie Fibronektin, Albumin, etc. angesehen. Durch gezielte Variation der physiko-chemischen Oberflächeneigenschaften von Mikropartikeln kann die Kinetik der Phagozytose durch die Zellen des MPS und das Ausmaß der Anreicherung der Partikel innerhalb der entsprechenden Organe (u.a. Leber, Lunge, Milz, Knochenmark) beeinflußt werden (passives targeting). Eine spezifische Anreicherung von Mikropartikeln in Zielgeweben oder Körperstrukturen, die nicht zu den Organen des RES gehören, ist auf diese Weise nicht möglich. Sie läßt sich vielmehr nur durch die Kombination der Mikropartikel mit Substanzen erzielen, die orts-, struktur- oder gewebespezifische Bindungseigenschaften besitzen (homing devices). Die bisher zur Anwendung in der Ultraschalldiagnostik beschriebenen Partikel eignen sich jedoch nur unzureichend als zur Kombination mit homing devices geeignete Präparationen.

So muß bei den in EP 0 458 079 und DE 38 03 972 beschriebenen Kontrastmitteln in Kauf genommen werden, daß sie nur mit Hilfe aufwendiger Verfahren hergestellt werden können, die die Verwendung organischer Lösungsmittel erforderlich machen, deren Einsatz aus Gründen des Umwelt- und Arbeitsplatzschutzes bedenklich ist. Zusätzlich muß vor Anwendung der Präparationen sichergestellt werden, daß die verwendeten organischen Lösungsmittel nicht mehr in dem pharmazeutisch zu verwendenden Produkt enthalten sind. Darüber hinaus sind zur Herstellung oberflächenaktive Hilfsstoffe (z.B. Tenside) notwendig, die bei Injektionspräparaten häufig als bedenklich angesehen werden. Weiterhin ist eine Steuerung des Anreicherungsverhaltens in verschiedenen Organen bei diesen Partikeln nicht steuerbar, eine Verknüpfung der Partikel der DE 38 03 972 mit sich selektiv anreichernden Verbindungen (sogenannten homing devices wie z.B. monoklonalen Antikörpern) ist nicht möglich.

Die in DE 40 04 430 beschriebenen Mikropartikel aus polymerisierten Aldehyden sind aufgrund der unklaren biologischen Abbaubarkeit ebenfalls nicht als Träger für substanz- oder strukturspezifische Substanzen geeignet. Ein weiterer Nachteil ist es, daß auch in diesem Falle zur Herstellung der Partikel oberflächenaktive Hilfsstoffe erforderlich sind.

Die in der EP 0 224 934 beschriebenen Mikropartikel aus Proteinen, insbesondere aus Albumin weisen eine nur sehr geringe *In vitro*- und *In vivo*-Stabilität auf.

Es war deshalb die Aufgabe der Erfindung, Mikropartikelpräparationen insbesondere für die Anwendung in der Ultraschalldiagnostik zu schaffen, die ohne den Einsatz physiologisch bedenklicher Lösungsmittel oder Hilfstoffe (z.B. Tenside) auskommen, leicht herstellbar und biologisch abbaubar sind, die entweder Substanzen mit orts- struktur- oder gewebespezifischen Bindungseigenschaften im Wandmaterial enthalten oder mit solchen kovalent verknüpft werden können und die eine ausreichende *In vitr*o- und *In vivo*-Stabilität aufweisen.

Erfindungsgemäß wird diese Aufgabe durch Mikrokapseln gelöst, deren Hülle aus der Kombination von Biopolymeren - ausgewählt aus der Gruppe der Polypeptiden (auch glykosilierten) oder der Collagenabbauprodukte - und synthetischem, während der Herstellung polymerisiertem Material gebildet wird.

Ein Gegenstand der Erfindung sind daher Mikrokapseln aus einem Copolymerisat aus mindestens einem synthetischen Polymeren und mindestens einem Biopolymeren, wobei sich als Biopolymere natürliche, synthetisch oder partialsynthetisch hergestellte oder gentechnologisch gewonnene Polypeptide wie z.B. Albumin, Collagenabbauprodukte, Gelatine, Fibrinogen, Fibronektin, Polygeline, Oxypolygelatine, deren Abbauprodukte sowie Poly-L-Lysin eignen. Die Biopolymere können auch glykosiliert sein. Als polymerisierbare Monomere eignen sich vorzugsweise Alkylcyanoacrylate, Acrylsäure, Acrylamid, Acrylsäurechlorid und Acrylsäureglycidester.

Die erfindungsgemäßen Mikropartikel eignen sich bei Herstellung in gasgesättigter Lösung durch den Einschluß des Gases besonders als Kontrastmittel für Ultraschalluntersuchungen. Sie wirken im Ultraschallfeld aufgrund des enthaltenen Gases als hocheffektive Streukörper. Desweiteren können sie durch diagnostischen Ultraschall zur Ausstrahlung eigenständiger Signale angeregt werden, welche z.B. mit Hilfe der Farbdopplertechnik ausgewertet werden können.

Als Gase kommen infrage Luft, Stickstoff, Kohlendioxid, Sauerstoff, Helium, Neon, Argon, Krypton oder deren Gemische. Die Beladung mit dem entsprechenden Gas oder Gasgemisch erfolgt durch Herstellung der Partikel in einer mit dem jeweiligen Gas oder Gasgemisch gesättigten wässrigen Lösung.

Die Mikropartikel können auch (gegebenenfalls zusätzlich) weitere mit Hilfe medizinisch-diagnostischer Verfahren, wie Magnetresonanztomographie, Magnetresonanzspektroskopie, Szintigraphie oder hochempfindlichen Magnetfeldmessungen mit geeigneten Magnetometern (Biomagnetismus) detektierbare Substanzen sowohl mikroverkapselt als auch im Wandmaterial als auch (gegebenenfalls mit Hilfe geeigneter Substanzen wie z.B. Chelatbildnern) an das Wandmaterial gekoppelt, enthalten. So ist es z.B. bei Verwendung radioaktiver Isotope möglich, die erfindungsgemäßen Mikropartikel in der Szintigraphie einzusetzen. Ebenso ist ihre Verwendung als Kontrastmittel in der Magnetresonanztomographie, Magnetresonanzspektroskopie oder bei Messungen des magnetischen Feldes durch die Mikroverkapselung oder Inkorporation in das Wandmaterial von geeigneten para-, superpara-, ferri- oder ferromagnetischen Substanzen möglich.

Überraschenderweise wurde gefunden, daß bei der Herstellung der erfindungsgemäßen Partikel (bei Einhaltung ausreichender Konzentrationen an Biopolymeren) der Zusatz von grenzflächenaktiven Substanzen, wie z.B. Tensiden nicht erforderlich ist. Dies stellt gegenüber den bisher bekannten Herstellungsverfahren für Mikropartikel auf der Basis von synthetischen Polymeren einen entscheidenen Vorteil dar, da die zur Erniedrigung der Grenzflächenspannung und zur Verhinderung der Partikelaggregation üblicherweise notwendigen Tenside als physiologisch bedenklich angesehen werden und deshalb vor der Anwendung im Organismus wieder bis auf verträgliche Restgehalte aus den Präparationen zu entfernen sind.

Als weiter Vorteil der erfindungsgemäßen Mikropartikelpräparationen sind die vielfältigen, dem jeweiligen Verwendungszweck anpaßbaren, Partikeleigenschaften zu nennen, die durch Variation verschiedener Herstellungsparameter leicht steuerbar sind. So können durch die Wahl des jeweils verwendeten Biopolymeren bzw. durch Veränderungen der funktionellen Gruppen des Biopolymeren (z.B. durch Acylierung mit Dicarbonsäureanhydriden, wie Bernsteinsäure-, Diglykolsäure-, Glutarsäure-, Maleinsäure- oder Fumarsäureanhydrid oder durch Acetylierung mit Monocarbonäureanhydriden, wie Essigsäureanhydrid oder Propionsäureanhydrid) die pharmakokinetischen Parameter der Mikropartikelpräparationen (Organverteilung Aufenthaltsdauer in der Blutstrombahn) beeinflußt werden.

Weiterhin kann der Gehalt des Biopolymeren im Wandmaterial in einem breiten Rahmen variiert werden, wodurch es möglich ist, die Zeitdauer des biologischen Abbaus des Kapselmaterials *in viv*o zu beeinflussen und dem gewünschten Verwendungszweck anzupassen. Dieser Gehalt läßt sich direkt über den Anteil des Biopolymeren in der Herstellungslösung steuern. So besteht beispielsweise das Wandmaterial von nach Beispiel 1 aus 1 % (V/V) Butylcyanoacrylsäure und 5 % Gelatine enthaltender autoklavierter wässriger Lösung hergestellter erfindungsgemäßer Mikropartikel zu 55 % (M/M) aus Biopolymeren, während bei gleichem Einsatz an Butylcyanoacrylat bei in 2,5 % iger wässriger autoklavierter Gelatinelösung hergestellten Mikropartikeln das Wandmaterial zu 35 % (M/M), bei in 7,5 % iger wässriger autoklavierter Gelatinelösung hergestellten Mikropartikeln das Wandmaterial zu 65 % (M/M) aus Biopolymeren besteht.

Überraschenderweise sind die erfindungsgemäßen Mikropartikel ohne Zusatz weiterer Hilfstoffe wie Laktose, Mannitol oder Sorbitol, wie sie üblicherweise zur Gefriertrocknung als Gerüstbildner eingesetzt werden, gefriertrockenbar. Diese Gerüstbildner sind nach der Trocknung für die mechanische Zerstörung eines erheblichen Teils der Mikrokapseln verantwortlich, der dann nicht mehr für die bildgebung nutzbar ist. Im Gegensatz dazu dient im Falle der erfindungsgemäßen Mikropartikel das im Überschuß eingesetzte Biopolymere des Wandmaterials als Gerüstbildner, wodurch überraschenderweise das Verhältnis von intakten zu zerstörten Mikrokapseln drastisch verbessert wird. Aufgrund dieses günstigeren Verhältnisses kann die zur Bildgebung erforderliche Dosis deutlich verringert werden.

Die erfindungsgemäßen Mikropartikel können jedoch auch - gegebenenfalls zusätzlich - pharmazeutische Wirkstoffe inkorporiert enthalten, indem z.B. das kontrastgebende Agens (im Fall von Kontrastmitteln für Ultraschalluntersuchungen handelt es sich dabei um ein Gas oder Gasgemische) und ein oder mehrere Wirkstoffe in den Partikeln mikroverkapselt werden. Vorzugsweise können die Wirkstoffe auch mit den für die orts-, struktur- oder gewebspezifischen Substanzen beschriebenen Methoden in das Wandmaterial inkorporiert werden. Falls es es sich bei den Wirkstoffen um Biopolymere handelt, können sie das Wandmaterial teilweise auch selbst bilden, indem sie bei der Herstellung entweder ausschließlich oder im Gemisch mit anderen geeigneten Biopolymeren (z.B. Gelatine, Albumin, Fibronektin, Poly-L-lysin) als Ausgangsmaterial zur Mikropartikelpräparation unter Zusatz eines polymeriserbaren Monomeren oder Oligomeren eingesetzt werden. Der besondere Vorteil der Kopplung von Wirkstoffen an den Biopolymeranteil des Kapselmaterials liegt darin, daß Wirkstoffe, die z.B. über Peptidbindungen an den Biopolymeranteil des Kapselmaterials gebunden sind, durch enzymatischen Abbau *in vivo* freigesetzt werden können.

Die erfindungsgemäßen Mikropartikel dienen insbesondere dem Nachweis oder der Therapie von Thrombosen und atherosklerotischen Veränderungen. Als besonders vorteilhaft ist dabei die Verwendung von Antikörpern oder Antikörperfragmenten gegen Fibrin, fibrinbindenden Plasmaproteinen oder deren Teilstrukturen, Gewebsplaminogenaktivator oder Teilstrukturen davon (z.B. Typ I-Homologie und Kringelsequenzen), Proteinbestandteilen von Lipoproteinen (auch Teilstrukturen) als homing devices anzusehen.

Weitere Anwendungsgebiete für die erfindungsgemäßen Mikropartikel können z.B. auch die Diagnose oder die Therapie hormoneller Funktionen sein (als besonders vorteilhaft ist hierbei die Verwendung von Peptidhormonen oder deren Abwandlungsprodukten mit der Fähigkeit zur Rezeptorbindung als homing devices anzusehen), oder die Diagnose oder Therapie von Läsionen von Blutgefäßendothelien (als besonders vorteilhaft ist hierbei entweder die Verwendung von Antikörpern bzw. Antikörperfragmenten gegen Substanzen aus der Integrin-Gruppe, insbesondere die Selectine wie z.B. LAM-1, ELAM-1 und GMP-140, oder die Verwendung von Rezeptoren bzw. deren bindungsvermittelnden Bruchstücke für Substanzen aus der Integrin-Gruppe, insbesondere die Selectine wie z.B. LAM-1, ELAM-1 und GMP-140, als homing devices anzusehen). Darüberhinaus können die erfindungsgemäßen Mikropartikel auch zur Diagnose oder Therapie von Tumoren genutzt werden, indem Antikörper oder Antikörpergemische gegen Oberflächenantigene von Tumoren als homing devices verwendet werden.

Die Herstellung der erfindungsgemäßen Mirkropartikel erfolgt durch die Polymerisation eines geeigneten reaktiven Monomeren oder Oligomeren (z.B. Cyanacrylsäurebutylester, Cyanacrylsäureisobutylester, Cyanacrylsäureisopropylester, Cyanacrylsäurepropylester, Cyanacrylsäureisohexylester, Cyanacrylsäurehexylester, Cyanacrylsäuremethylester, Acrylsäure, Acrylamid, Acrylsäureglycidester, Acrylsäurechlorid) in einer Konzentration bezogen auf das Gesamtvolumen der Herstellungslösung von 0,01 - 10 % (m/V) (vorzugsweise 0,1 - 10 %) unter geeigneten Bedingungen (z.B. durch Wahl des pH, durch Zusatz von Radikalen, und durch UV-Einstrahlung) unter Dispergieren in wässriger Phase, die ein Biopolymer, z.B. Albumin, Gelatine, Oxypolygelatine, Polygeline, Fibronektin, Poly-L-Lysin in einer Konzentration von 0,5 - 20 % (m/V) (vorzugsweise 1 % - 15 % (m/V)) gelöst enthält. Bei Verwendung von Collagenabbauprodukten wie z. B. Gelatine, Polygeline oder Oxypolygelatine ist es häufig vorteilhaft, die Lösungen vor der Mikropartikelherstellung zu autoklavieren. Nach beendeter Polymerisation werden die entstandenen Mikropartikel je nach Dichte und Partikelgröße durch einmalige oder mehrmalige Zentrifugation, Filtration oder Flotation abgetrennt, gegebenenfalls durch Dialyse weiter gereinigt und in einem physiologisch verträglichen Suspensionsmittel (vorzugsweise Wasser für Injektionszwecke) bis zur gewünschten Konzentration suspendiert. Die Suspensionen können durch den Zusatz geeigneter wasserlöslicher Stoffe wie z.B. Glucose, Mannitol, Sorbitol, Kochsalz, Galactose, Laktose, Fruktose, Trehalose isotonisiert werden.

Die Größenverteilung der bei der Herstellung entstehenden Mikropartikel ist durch die Art des verwendeten Rührgerätes und die Umdrehungszahl in weiten Bereichen steuerbar.

Die Herstellung gasgefüllter Mikropartikel erfolgt, indem die Reaktion in einer mit dem gewünschten Gas gesättigten Lösung durchgeführt wird. Dabei ist die Dichte der resultierenden Mikropartikel, d.h. das Verhältnis zwischen Wandmaterial und Gasanteil, sowohl durch die Rührbedingungen als auch insbesondere durch den Anteil an Biopolymeren beim Herstellungsprozess steuerbar.

Sollen Mikropartikel erhalten werden, bei denen der Kern aus demselben Material wie die Hülle besteht, so ist bei der Herstellung darauf zu achten, daß durch die Wahl eines geeigneten Rührgerätes und einer geeigneten Rührgeschwindigkeit ein Schäumen der Reaktionslösung vermieden wird.

Die geforderte Kombinierbarkeit mit orts-, struktur- oder gewebsspezifischen Substanzen, die eine zusätzliche Anreicherung der Mikropartikel in Zielgebieten außerhalb der Organe des RES sicherstellen sollen (homing devices), erfolgt entweder über die vor der Mikropartikelpräparation oder nachträglich durchgeführte Kopplung der Substanzen an die das Hüllmaterial mitbildenden Polypeptide mit bekannten Methoden der Biochemie zur Kopplung von Aminosäuren (z.B. W. König, R. Geiger: Eine neue Methode zur Synthese von Peptiden: Aktivierung der Carboxylgruppe mit Dicyclohexylcarbodiimid unter Zuatz von 1-Hydroxy-benzotriazolen. Chem. Ber. 103, 788-798 (1970)), oder dadurch, daß die Mikropartikel in einer wässrigen Lösung der orts-, struktur- oder gewebsspezifischen Substanz hergestellt werden, falls diese ein Polypeptid darstellt, so daß die Substanz direkt als Bestandteil des Hüllmaterials dient.

Als an die Mikropartikel koppelbare, oder das Hüllmaterial mitbildende orts-, struktur-, oder gewebsspezifische Substanzen kommen vorzugsweise Antikörper, konjugierte Antikörper, Hormone (insbesondere Peptidhormone), Transferrin, Fibronektin, Heparin, Transcobalamin, epidermaler Wachstumsfaktor, Lipoproteine, Plasmaproteine sowie deren spezifitätsvermittelnden Teilstrukturen und Oligopeptide wie RGD, RGDS, RGDV und RGDT in Betracht.

Als an die Mikropartikel koppelbare chelatisierende Liganden kommen Diethylentriaminpentaessigsäure oder deren Derivate infrage. Die Verknüpfung dieser Liganden mit den Partikeln erfolgt in an sich bekannter Weise [Hanatowich et al., Science 220 (1983) 613]. Anschließend werden die Partikel mit den den gewünschten Metallionen zu dem jeweiligen partikelfixierten Metallkomplex umgesetzt.

Die Wahl des verwendeten Metallions richtet sich nach dem gewünschten Anwendungsbereich. Im Bereich der NMR-Diagnostik werden erfindungsgemäß bevorzugt paramagnetische Metallionen der Elemente der Ordnungszahlen 21-29 und 57-70, insbesondere Gadolinium(III)ionen, eingesetzt. Für die Anwendung in der Szintigraphie finden geeignete Emitter radioaktiver Strahlung, bevorzugt ¹¹¹In oder ^{99m}Tc, ¹²³I und ¹³¹I Anwendung.

Die fertigen Mikropartikelsuspensionen können direkt für den jeweiligen vorbestimmten Verwendungszweck eingesetzt werden, jedoch hat es sich zur Verbesserung der Lagerstabilität als vorteilhaft erwiesen, die Suspensionen unter Zusatz von Gerüstbildnern (wie z.B. Trehalose, Polyvinylpyrrolidon, Laktose, Mannitol, Sorbitol, Glycin), die auch zur Einstellung der Tonizität dienen können, einzufrieren und anschließend gefrierzutrocknen. Als besonders vorteilhaft hat es sich erwiesen das im Überschuß eingesetzte Biopolymere selbst als Gerüstbildner einzusetzen. In beiden Fällen ist es zweckmäßig, die Suspensionen während des Einfrierens zu bewegen, um ungleiche Partikelverteilungen im gefrorenen Gut durch Sedimentation oder Flotation zu verhindern. Die Herstellung der gebrauchsfertigen, injizierbaren Suspensionen aus den gefriergetrockneten Zubereitungen, erfolgt durch Resuspendieren des Lyophilisats in einem pharmazeutisch akzeptablen Suspensionsmedium wie z.B Wasser p.i., wäßrige Lösungen eines oder mehrerer anorganischer Salze wie physiologische Elektrolyt-Lösungen, wäßrige Lösungen von Mono- oder Disacchariden wie Glucose oder Lactose, Zuckeralkoholen wie Mannit enthalten, bevorzugt jedoch in für Injektionszwecke geeignetem Wasser. Die Gesamtkonzentration der gegebenenfalls gelösten Stoffe beträgt 0-20 Gewichts-Prozent.

Die Konzentration des gebrauchsfertigen Kontrastmittels kann im Bereich von 0,01 bis 20 mg, bevorzugt von 0,5 bis 6 mg Partikel/ml Suspensionsmedium eingestellt werden. Die injizierte Dosis ist abhängig vom jeweiligen Verwendungszweck; sie liegt bei ultraschalldiagnostischen Untersuchungen bei der Untersuchung der Gefäße im Bereich 1 bis 500 µg, bevorzugt zwischen 10 und 100 µg Partikel/kg Körpergewicht, bei der Untersuchung von Leber und Milz mittels Farbdopplersonographie im Bereich von 50 bis 1000, bevorzugt zwischen 200 und 600 µg/kg Körpergewicht. Die Erfindung wird durch die nachfolgenden Beispiele erläutert:

### Beispiel 1

In 300 ml destilliertem Wasser werden 15 g Gelatine (300 Bloom) gelöst und mit Salzsäure auf pH 3,0 eingestellt. Die Lösung wird 30 Minuten bei 121 °C autoklaviert. Nach dem Erkalten auf Raumtemperatur wird der pH-Wert der Lösung zu pH 5,0 korrigiert (mit Natronlauge) und in einem 2000 ml Becherglas mit einem schnellaufenden Rührer bei 10000 Upm gerührt. Zu der Lösung werden unter Rühren 3 ml Cyanacrylsäurebutylester langsam (10 Minuten) zugetropft. Die entstehenden Mikropartikel werden 60 Minuten weitergerührt. Danach wird die Suspension in einem Scheidetrichter 2 d flotiert. Der Unterstand wird abgelassen und der Überstand wird mit destilliertem Wasser zu 100 ml ergänzt. Die Suspension enthält gasgefüllte, schallaktive Mikropartikel in einer Größe von ca. 0,1 - 8 µm, wobei durch zusätzliche Flotation oder Filtration erforderlichenfalls die Teilchengrößen weiter eingeengt werden können (z.B. auf 0,5 - 3 µm). Die Kapselwand der Mikropartikelartikel besteht zu ca. 55 % (M/M) aus Polypeptiden und zu ca. 45 % (M/M) aus Polycyanacrylsäurebutylester. Die Partikel sind ohne den Zusatz grenzflächenaktiver Hilfsstoffe in Wasser dispergierbar. Sie neigen nicht zur Aggregation. Durch den Zusatz eines geeigneten Hilfsstoffes (z.B. Glucose, Natriumchlorid, Mannitol, Laktose, Galaktose) kann die Suspension isotonisiert werden.

Die Suspension läßt sich erforderlichenfalls zur Erhöhung der Lagerstabilität ohne Verlust ihrer Eignung als Kontrastmittel für Ultraschalluntersuchungen vorzugsweise nach Zusatz eines Kryoprotektors, wie z.B. Laktose, Polyvinylpyrolidon, Mannitol, Glycin gefriertrocknen.

### Beispiel 2

500 mg Poly-L-Lysin (MG 5000) werden in 20 ml destilliertem Wasser gelöst und mit Phosphatpuffer auf pH 4.5 eingestellt. 100 mg Acrylsäureglycidester dazugegeben, das Gemisch wird mit einem schnelllaufenden Rührer unter Kühlung bei 20 °C gerührt. Es werden 10 mg Ammonumperoxydisulfat und 0,1 ml N,N,N',N'-Tetramethylendiamin zugegeben. Es wird für weitere 90 Minuten gerührt. Die entstandenen gasgefüllten Mikropartikel werden durch Flotation abgetrennt. Die Teilchengröße der Mikropartikel liegt zwischen 0,2 und 6 µm.

### Beispiel 3

7,5 g Polygeline werden in 200 ml Wasser für Injektionszwecke gelöst. Die Lösung wird mit Phosphorsäure auf pH 3,0 eingestellt und mit Wasser für Injektionszwecke zu 300 ml ergänzt. Die Lösung wird durch einen 0,22 µm Filter zur Sterilfiltration filtriert und mit einem schnelldrehenden Dissolver bei 6000 Upm gerührt. Unter Rühren werden langsam eine Mischung aus 1.5 ml Cyanacrylsäureisopropylester und 1,5 ml Cyanacrylsäurebutylester zugetropft. Es wird 120 Minuten lang weitergerührt. Die entstandene Suspension wird drei Tage im Scheidetrichter flotiert. Das weitere Vorgehen entspricht Beispiel 1. Die entstandenen Mikropartikel enthalten Gas. Sie eignen sich als Kontrastmittel für Ultraschalluntersuchungen. Ihr Wandmaterial besteht zu ca. 22 % (M/M) aus Biopolymer, zu ca. 40 % (M/M) aus Polycyanoacrylsäurebutylester und zu ca. 38 % (M/M) aus Polycyanoacrylsäureisopropylester. Sie lassen sich ohne Zusatz grenzflächenaktiver Hilfsstoffe in Wasser dispergieren ohne dabei zu aggregieren. Ihre Teilchengröße beträgt ca. 0,2 - 6 µm.

### Beispiel 4

10 g Humanalbumin werden in 200 ml Wasser für Injektionszwecke gelöst. Die Lösung wird mit Salzsäure auf pH 4,0 eingestellt und mit Wasser für Injektionszwecke zu 300 ml ergänzt. Die Lösung wird durch einen 0,22 µm Filter zur Sterilfiltration filtriert und mit einem schnelldrehenden Dissolver bei 10000 Upm gerührt. Unter Rühren werden langsam 2 ml Cyanacrylsäureisopropylester zugetropft. Es wird 60 Minuten lang weitergerührt. Die entstandene Suspension wird drei Tage im Scheidetrichter flotiert. Das weitere Vorgehen entspricht Beispiel 1. Die entstandenen Mikropartikel enthalten Gas. Sie eignen sich als Kontrastmittel für Ultraschalluntersuchungen. Ihr Wandmaterial besteht zu ca. 30 % (M/M) aus Humanalbumin und zu ca. 70 % (M/M) aus Polycyanoacrylsäureisopropylester. Sie lassen sich ohne Zusatz grenzflächenaktiver Hilfsstoffe in Wasser dispergieren ohne dabei zu aggregieren. Ihre Teilchengröße beträgt durchschnittlich ca. 0,2 - 3 µm.

### Beispiel 5

250 ml Oxypolygelatine-Lösung werden mit Salzsäure auf pH 2,5 eingestellt und mit Wasser für Injektionszwecke zu 300 ml ergänzt. Die Lösung wird durch einen 0,22 µm Filter zur Sterilfiltration filtriert und mit einem schnelidrehenden Rotor-Stator-Rührer bei 8000 Upm gerührt. Unter Rühren werden langsam 3 ml Cyanacrylsäurebutylester zugetropft. Es wird 90 Minuten lang weitergerührt. Die entstandene Suspension wird drei Tage im Scheidetrichter flotiert. Das weitere Vorgehen entspricht Beispiel 1. Die entstandenen Mikropartikel enthalten Gas. Sie eignen sich als Kontrastmittel für Ultraschalluntersuchungen. Ihr Wandmaterial besteht zu ca. 25 % (M/M) aus Oxypolygelatine und zu ca. 75 % (M/M) aus Polycyanoacrylsäurebutylester. Sie lassen sich ohne Zusatz grenzflächenaktiver Hilfsstoffe in Wasser dispergieren ohne dabei zu aggregieren. Ihre Teilchengröße beträgt ca. 0,2 - 4 µm.

### Beispiel 6

500 mg Fibronektin werden in 5 ml destilliertem Wasser gelöst und mit Salzsäure auf pH 3,5 eingestellt. Die Lösung wird durch einen 0,22 µm Filter zur Sterilfiltration filtriert und mit einem schnelldrehenden Rotor-Stator-Rührer in einem gekühlten 15 ml Gefäß (15 °C) bei 8000 Upm gerührt. Unter Rühren werden langsam 0,3 ml Cyanacrylsäurebutylester zugetropft. Es wird 90 Minuten lang weitergerührt. Die entstandene Suspension wird drei Tage im Scheidetrichter flotiert. Der Überstand wird in 2 ml Wasser für Injektionszwecke, welches 100 mg Mannitol enthält, suspendiert. Die Suspension wird bei -50 °C unter Schütteln eingefroren und gefriergetrocknet. Vor Anwendung werden die Mikropartikel mit 2 ml Wasser für Injektionszwecke redispergiert. Die Teilchengröße der Mikropartikel beträgt durchschnittlich 0,8 µm. Sie sind als Kontrastmittel für Ultraschalluntersuchungen geeignet. Das Wandmaterial der Mikropartikel besteht zu ca. 35 % (M/M) aus Fibronektin und zu ca 65 % (M/M) aus Polycyanoacrylsäurebutylester.

### Beispiel 7

100 mg eines Antikörpers gegen Fibrin werden in 4 ml Phosphatpuffer (pH 4,5) gelöst. Die Lösung wird durch einen 0,22 µm Filter zur Sterilfiltration filtriert und in einem doppelwandigen Rührgefäß (Fassungsvolumen 10 ml) mit einem schnelldrehenden Dissolver-Rührer unter Kühlung bei 6000 Upm gerührt. Während des Rührens werden langsam 0,2 ml Cyanacrylsäurebutylester zugetropft. Es wird 60 Minuten lang weitergerührt. Die entstandene Suspension wird zwei Tage im Scheidetrichter flotiert. Der Unterstand wird abgelassen. der Überstand wird mit 200 mg Laktose und 2 ml Wasser für Injektionszwecke versetzt. Die Suspension wird unter Schütteln im Kältebad bei -40 °C eingefroren und anschließend gefriergetrocknet. Vor Anwendung werden die Mikropartikel mit 2 ml Wasser für Injektionszwecke resuspendiert. Sie sind gasgefüllt und eignen sich als Kontrastmittel für Ultraschalluntersuchungen. Ihre Teilchengröße beträgt durchschnittlich ca. 1 µm. Das Wandmaterial der Mikropartikel besteht zu ca. 20 % (M/M) aus dem Antikörper und zu ca. 80 % (M/M) aus Polycyanoacrylsäurebutylester.

### Beispiel 8

15 g Polygeline werden in 50 ml Wasser für Injektionszwecke gelöst, unter pH-Kontrolle wird tropfenweise 2 N Natronlauge zugegeben. Es werden insgesamt 2 g Diglykolsäureanhydrid successive zugegeben, wobei der pH-Wert zwischen 7,5 und 8,0 gehalten wird. Nach Beendigung der Reaktion wird die überschüssige Diglykolsäure durch mehrfache Ultrafiltration (Ausschlußgrenze MG 1000) aus der Lösung entfernt. Die acylierte Polygelinelösung wird mit Wasser für Injektionszwecke auf 300 ml ergänzt und durch einen 0,22 µm Filter filtriert. Unter Rühren bei 10000 Upm werden langsam 3 ml Cyanacrylsäurebutylester zugesetzt. Nach beendeter Zugabe wird 60 Minuten lang weitergerührt. Die entstandenen gasgefüllten Mikropartikel werden durch Zentrifugation bei 1500 Upm (30 Minuten) abgetrennt und in 50 ml Wasser für Injektionszwecke aufgenommen. Sie sind ohne Zusatz oberflächenaktiver Hilfsstoffe in Wasser dispergierbar ohne zu aggregieren. Ihre Teilchengröße liegt bei 0,1 - 6 µm. Das Wandmaterial der Mikropartikel besteht zu ca. 45 % (M/M) aus acylierter Polygeline und zu ca. 55 % (M/M) aus Polycyanoacrylsäurebutylester.

### Beispiel 9

20 ml der nach Beispiel 3 hergestellten gasenthaltenden Mikropartikel werden in 20 ml Phosphatpuffer pH 4,5 aufgenommen. Die Suspension wird bei 4 °C gerührt (100 Upm) und es werden 25 mg (3-Dimethylaminopropyl)N'-ethylcarbodiimid-HCl dem Gemisch hinzugefügt. Nach 60 Minuten werden 25 mg Fibronektin, die zuvor in 10 ml Phosphatpuffer gelöst wurden, zu der Mikropartikelsuspension gegeben. Es wird über 60 Minuten bei 4 °C und weitere 120 Minuten bei Raumtemperatur gerührt. Anschließend wird die Suspension dreifach gegen Phosphatpuffer pH 4,5 dialysiert (Ausschlußgrenze MG 1000) und zwei Tage im Scheidetrichter flotiert. Der Überstand wird in 20 ml Wasser für Injektionszwecke aufgenommen, mit 5 % Polyvinylpyrrolidon (m/V) versetzt, bei -40 °C unter Schütteln eingefroren und gefriergetrocknet.

### Beispiel 10

Das Lyophilisat aus Beispiel 9 wird mit 20 ml 5 % iger Glukoselösung resuspendiert. 0,1 ml davon werden zu 10 ml PBS-Lösung von 37 °C gegeben, die einen frisch hergestellten Fibrinclot enthält (Durchmesser 1 mm). Nach 10 minütiger Inkubation unter Schütteln im Wasserbad wird der Clot entnommen, fünfach mit je 10 ml PBS (pH 7,4) gewaschen und anschließend sonographisch untersucht. Im Farbdoppler lassen sich eindeutig Signale anhängender Mikropartikel nachweisen. Mit den Partikeln aus Beispiel 3 (ohne die in Beispiel 9 gezeigte Umsetzung mit Fibronektin) wird analog verfahren. Bei der sonographischen Untersuchung der Clots lassen sich (auch im Farbdoppler) keine anhängenden Mikropartikel nachweisen.

### Beispiel 11

5 ml der nach Beispiel 3 hergestellten gasenthaltenden Mikropartikel werden in 5 ml Phosphatpuffer pH 4,5 aufgenommen. Die Suspension wird bei 4 °C gerührt (100 Upm), und es werden 10 mg (3-Dimethylaminopropyl)-N'-ethylcarbodiimid-HCl dem Gemisch hinzugefügt. Nach fünf Minuten werden 2,5 mg eines Antikörpers gegen Fibrin (Nr. 0541 clone E8, Immunotech, Marseille, Frankreich), die zuvor in 1 ml Phosphatpuffer gelöst wurden, zu der Mikropartikelsuspension gegeben. Es wird 60 Minuten bei 4 °C und weitere 120 Minuten bei Raumtemperatur gerührt. Anschließend wird die Suspension dreifach gegen Phosphatpuffer pH 4,5 dialysiert (Ausschlußgrenze MG 1000) und zwei Tage im Scheidetrichter flotiert. Der Überstand wird in 2 ml Wasser für Injektionszwecke aufgenommen, mit 5 % Polyvinylpyrrolidon (m/V) versetzt, bei -40 °C unter Schütteln eingefroren und gefriergetrocknet.

### Beispiel 12

Das Lyophilisat aus Beispiel 11 wird mit 2 ml 5 % iger Glukoselösung resuspendiert. 0,1 ml davon werden zu 10 ml PBS-Lösung von 37 °C gegeben, die einen frisch hergestellten Fibrinclot enthält (Durchmesser 1 mm). Nach 10 minütiger Inkubation unter Schütteln im Wasserbad wird der Clot entnommen, fünf Mal mit je 10 ml PBS (pH 7,4) gewaschen und anschließend sonographisch untersucht. Im Farbdoppler lassen sich eindeutig Signale anhängender Mikropartikel nachweisen. Mit den Partikeln aus Beispiel 3 (ohne die in Beispiel 11 gezeigte Umsetzung mit dem Antikörper gegem Fibrin) wird analog verfahren. Bei der sonographischen Untersuchung der Clots lassen sich (auch im Farbdoppler) keine anhängenden Mikropartikel nachweisen.

### Beispiel 13

0,1 ml der resuspendierten Partikel aus Beispiel 6 werden im Versuchsaufbau analog Beispiel 11 auf ihre Fibrinbindung geprüft. In der sonographischen Untersuchung lassen sich deutlich an den Clot gebundene Mikropartikel nachweisen.

### Beispiel 14

0,1 ml der resuspendierten Partikel aus Beispiel 7 werden im Versuchsaufbau analog Beispiel 11 auf ihre Fibrinbindung geprüft. In der sonographischen Untersuchung lassen sich deutlich an den Clot gebundene Mikropartikel nachweisen.

### Beispiel 15

10 ml der nach Beispiel 8 hergestellten Mikropartikel werden in 10 ml Phosphatpuffer pH 4,5 aufgenommen, und es werden 20 mg 1-Hydroxybenzotriazol hinzugefügt. Nach Abkühlen auf 4 °C wird gerührt (100 Upm), und es werden 10 mg (3-Dimethylaminopropyl)-N'-ethylcarbodiimid-HCl zugegeben. Es wird 60 Minuten lang bei 4 °C weitergerührt. Danach wird für weitere 60 Minuten bei Raumtemperatur gerührt. Zu der Suspension werden bei Raumtemperatur 10 mg Pankreozymin, die zuvor in 5 ml Phosphatpuffer gelöst wurden, gegeben. Es wird über 120 Minuten bei Raumtemperatur gerührt. Anschließend wird die Suspension fünf Mal gegen Phosphatpuffer pH 4,5 dialysiert (Ausschlußgrenze MG 1000) und zwei Tage im Scheidetrichter flotiert. Der Überstand wird in 10 ml Wasser für Injektionszwecke aufgenommen, mit 5 % Polyvinylpyrrolidon (m/V) versetzt, bei -40 °C unter Schütteln eingefroren und anschließend gefriergetrocknet.

### Beispiel 16

Das Lyophilisat aus Beispiel 15 wird mit 10 ml Wasser für Injektionszwecke resuspendiert. 0,1 ml der Suspension werden einer Ratte in die Schwanzvene injiziert. Nach 10 Minuten wird das Pankreas entnommen und im Wasserbad sonographisch untersucht. Im Farbdoppler sind Ultraschallsignale der Mikropartikel zu erkennen.

### Beispiel 17

5 ml der nach Beispiel 3 hergestellten gasenthaltenden Mikropartikel werden in 5 ml Phosphatpuffer pH 4,5 aufgenommen. Die Suspension wird bei 4 °C gerührt (100 Upm), und es werden 10 mg (3-Dimethylaminopropyl)-N'-ethylcarbodiimid-HCl dem Gemisch hinzugefügt. Nach fünf Minuten werden 5 mg tPA, die zuvor in 1 ml Phosphatpuffer gelöst wurden, zu der Mikropartikelsuspension gegeben. Es wird über 24 Stunden bei 4 °C weitergerührt. Anschließend wird die Suspension dreifach gegen Phosphatpuffer pH 4,5 dialysiert (Ausschlußgrenze MG 1000) und zwei Tage im Scheidetrichter flotiert. Der Überstand wird in 2 ml Wasser für Injektionszwecke aufgenommen.

### Beispiel 18

Es werden zwei Fibrinclots (Masse je ca. 50 mg) hergestellt, die in 20 ml Plasma gegeben werden. Zu den Clots werden 0,05 ml der nach Beispiel 17 hergestellten Partikelsuspension gegeben. Nach 10 Minuten werden die Clots aus dem Plasma entnommen, in der sonographischen Untersuchung zeigen sich im Farbdoppler Signale anhängender Mikropartikel.

### Beispiel 19

In 20 ml einer wässrigen Suspension aus Magnetitpartikeln (ca. 20 mmol Eisen/ml, Durchmesser der Partikel ca. 20 nm) werden 0,6 g Gelatine gelöst. Die Lösung wird mit Salzsäure auf ph 3 eingestellt. Unter Rühren (3000 Upm) werden 0,2 ml Cyanacrylsäureisobutylester langsam zugesetzt. Nach beendeter Zugabe wird 90 Minuten lang weitergerührt. Die Suspension wird zentrifugiert (2000 Upm, 60 Minuten). Der Überstand wird verworfen, der Unterstand wird in 10 ml PBS pH 7,4 (10 mmol) aufgenommen. Die Suspension wird auf 4 °C abgekühlt und es werden unter Rühren (100 Upm) 10 mg (3-Dimethylaminopropyl)-N'-ethylcarbodiimid-HCl zugegeben. Es wird 60 Minuten lang bei 4 °C weitergerührt. Danach werden 5 mg eines Antikörpers gegen Fibrin (Nr. 0541 clone E8, Immunotech, Marseille, Frankreich) zugegeben. Es wird 60 Minuten bei 4 °C und anschließend 120 Minuten bei Raumtemperatur weitergerührt. Die Suspension wird fünf Mal gegen PBS pH 7,4 (10 mmol) ultrafiltriert (Ausschlußgrenze MG 5000). Danach wird die Suspension zentrifugiert (2000 Upm, 60 Minuten). Der Unterstand wird in 5 ml Wasser für Injektionszwecke aufgenommen, das 5 % Mannitol (m/V) enthält und durch einen 5 µm Membranfilter filtriert. Das Filtrat wird bei -40 °C eingefroren und anschließend gefriergetrocknet.

### Beispiel 20

15 g Gelatine (300 Bloom) werden in 150 ml Wasser für Injektionszwecke bei 80 °C gelöst. Nach dem Erkalten wird die Lösung mit 0,1 N HCl auf pH 2,5 eingestellt und mit Wasser für Injektionszwecke zu 300 ml ergänzt. Die Lösung wird autoklaviert (Verfahren A 121, Deutsches Arzneibuch 9. Ausgabe). Der pH-Wert der autoklavierten Lösung wird kontrolliert und erforderlichenfalls zu pH 2,5 korrigiert. Zu der Lösung werden unter Rühren 3 ml Cyanacrylsäureisobutylester gegeben. Es wird 90 Minuten weitergerührt. Die entstandene Mikropartikelsuspension wird bei 1000 Upm 60 min lang zentrifugiert, der Überstand in 50 ml Wasser für Injektionszwecke aufgenommen und erneut bei 1000 Upm 60 min lang zentrifugiert. Dies wird insgesamt 5 mal wiederholt. Der Überstand der letzten Zentrifugation wird in 50 ml PBS (pH 7,0) aufgenommen und unter Rühren zu 0,1 mg festem Diethylentriaminpentaessigsäuredianhydrid (vgl.: Hnatowich et al (1983) Science 220:613) gegeben. Es wird 5 Minuten gerührt. Die Suspension wird bei 1000 Upm 60 min lang zentrifugiert, der Überstand in 50 ml Wasser für Injektionszwecke aufgenommen. Die Zentrifugation gegen Wasser für Injektionszwecke wird noch 4 mal wiederholt. Der Überstand der letzten Zentrifugation wird mit 50 ml Wasser für Injektionszwecke aufgenommen und durch eine Filterkolonne aus HDC-Porenfiltern der Porengrößen 70, 40, 20 und 10 µm filtriert. Das Filtrat enthält ca 2 x 10⁹ Partikel/ml, die DTPA-Gruppen auf ihrer Oberfläche besitzen. Die mittlere Partikelgröße beträgt ca. 2 µm. Die Partikel lassen sich mit den bekannten Methoden mit radioaktiven Metallionen (z.B. In-111 oder Tc-99) markieren.

### Beispiel 21

Fibrinclot wie Beispiele zur Schallanwendung, nur mit Gammacounter statt mit Doppler Nachweis führen.

### Beispiel 22

7,5 g Polygeline werden in 150 ml Wasser für Injektionszwecke bei 80 °C gelöst. Nach dem Erkalten wird die Lösung mit 0,1 N HCl auf pH 3 eingestellt und mit Wasser für Injektionszwecke zu 300 ml ergänzt. Die Lösung wird autoklaviert (Verfahren A 121, Deutsches Arzneibuch 9. Ausgabe). Der pH-Wert der autoklavierten Lösung wird zu pH 2 korrigiert. Zu der Lösung werden unter Rühren 3 ml Cyanacrylsäurebutylester gegeben. Es wird 90 Minuten weitergerührt. Die entstandene Mikropartikelsuspension wird bei 1000 Upm 60 min lang zentrifugiert, der Überstand in 50 ml Wasser für Injektionszwecke aufgenommen und erneut bei 1000 Upm 60 min lang zentrifugiert. Dies wird insgesamt 5 mal wiederholt. Der Überstand der letzten Zentrifugation wird in 50 ml PBS (pH 7,4) aufgenommen und auf 4 C abgekühlt. Unter Rühren bei 4 °C werden 50 mg Streptavidin und 5 mg EDC zugegeben. Es wird 1 h weitergerührt. Die Suspension wird 3 mal zentrifugiert (1000 Upm, 60 min). Nach jeder Zentrifugation wird der Überstand mit 50 ml PBS (pH 7,0, 10mM Phosphat) aufgenommen. Der Antikörper gegen Fibrin wird in einem molaren Verhältnis von 1:5 mit sulfosuccinimidyl-6-(biotinamido)-hexanoat (NHS-LC-Biotin) nach der Methode von DJ Hnatovitch et al, J. Nucl. Med. 28 (1987) 1294-1302 markiert.

### Beispiel 23

Es werden 0,5 mg des Biotin-markierten Antikörpers gegen Fibrin aus Beispiel 22 einem mit einer Cholesterin enthaltenden Diät gefütterten Kanninchen intravenös injiziert. Nach 3 Stunden werden die Partikel aus Beispiel 22 nachinjiziert. 10 Minuten später wird dem zuvor getötetem Tier die Halsaterie entnommen und es werden die atherosklerotischen Arterienabschnitte im Wasserbad im Dopplermodus auf Kontrastmittelsignale untersucht. Es lassen sich eindeutige Schallsignale von anhaftenden Partikeln nachweisen.

### Beispiel 24

a) 20 ml der nach Beispiel 8 hergestellten Mikropartikelsuspension werden mittels 5 ml Phosphatpuffer auf einen pH-Wert von 4,5 gebracht und anschließend mit 50 mg eines 125-Iod markierten Antikörpers gegen Fibrin (5 µCi) versetzt. Unter Rühren bei 4 °C werden, zu der Reaktionsmischung 500 mg (3-Dimethylaminopropyl)-N 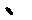 -ethylcarbodiimid-Hydrochlorid gegeben. Anschließend wird 8 Stunden unter Kühlung weitergerührt, die Mikropartikel durch Zentrifugation abgetrennt und insgesamt dreimal mit je 20 ml Wasser für Injektionszwecke gewaschen, wobei jeder einzelne Waschvorgang in der Weise erfolgt, daß die Partikel in Wasser resuspendiert und anschließend zentrifugiert werden. Nach dem letzten Waschvorgang werden die Partikel in 20 ml Wasser für Injektionszwecke resuspendiert.
   Der Bindungsgrad des Antikörpers auf dem Partikel wird mit einem Gamma-Counter anhand der 125-Jod Aktivität bestimmt. Danach sind 93 % der ursprünglich eingesetzten Antikörpermenge fest an die Partikeloberfläche gebunden.
b) Mikropartikel hergestellt nach Beispiel 4 der DE 38 03 972 werden in einer dem Beispiel 24 a) entsprechenden Menge mit 50 mg eines 125-Jod markierten Antikörpers gegen Fibrin (5 µCi) unter sonst identischen Reaktionsbedingungen umgesetzt.
   Ein Vergleich des Bindungsgrad mit den Partikeln nach Beispiel 24 a) zeigt einen erheblich geringeren Wert von nur 1 %.

## Patentansprüche

1. Mikrokapseln für die Diagnose und/oder Therapie aus biologisch abbaubaren Polymeren, dadurch gekennzeichnet, daß
das Wandmaterial aus einem Mischpolymeren aus mindestens einem synthetischen polymeren Material und mindestens einem Biopolymeren aus
(i) der Gruppe der Polypeptide, welche gegebenenfalls glykosiliert sind oder
(ii) der Gruppe der Collagenabbauprodukte, besteht, wobei das Biopolymer
a) orts-, struktur- oder gewebespezifische Eigenschaften aufweist oder
b) funktionelle Gruppen besitzt, über welche gegebenenfalls chelatisierende Liganden oder deren Metallkomplexe und/oder orts-, struktur- oder gewebespezifische Substanzen gebunden sind,
und das Wandmaterial gegebenenfalls zusätzlich einen oder mehreren pharmazeutischer(n) Wirkstoff(en) enthält
und das der Kern der Kapseln aus
a) einem Gas oder Gasgemischen und/oder
b) einem pharmazeutischen Wirkstoff oder Wirkstoffgemisch oder
c) dem gleichen Material wie die Kapselwand besteht,
unter den Maßgaben, daß das synthetische polymere Material nicht aus polymerisierbaren Aldehyden aufgebaut ist, daß das Gewichtsverhältnis vom Biopolymer zum synthetischen Polymer im Bereich von 10:90 bis 80:20 liegt und das die Mikrokapselgröße 0,5 bis 8 µm beträgt.

2. Mikrokapseln gemäß Anspruch 1, dadurch gekennzeichnet, daß das synthetische Polymere aus monomerer Acrylsäure, Acrylamid, Acrylsäurechlorid, Acrylsäureglycidester oder monomeren Alkylcyanoacrylaten aufgebaut wird.

3. Mikrokapseln gemäß Anspruch 1, dadurch gekennzeichnet, daß das Biopolymer Albumin, Fibrinogen, Fibronektin, Gelatine, Polygeline, Oxypolygelatine oder Poly-L-Lysin ist.

4. Mikrokapseln gemäß Anspruch 1, dadurch gekennzeichnet, daß die gegebenfalls über die funktionellen Gruppen des Biopolymeren gebundene orts-, struktur und gewebespezifische Substanzen Antikörper, konjugierte Antikörper, Hormone, Transferrin, Fibronektin, Heparin, Transcobalamin, epidermaler Wachstumsfaktor, Lipoproteine, Plasmaproteine, Peptide oder Oligopeptide sind, die bevorzugt die Aminosäuresequenzen RGD, RGDS, RGDV oder RGDT enthalten.

5. Mikrokapseln gemäß Anspruch 1, dadurch gekennzeichnet, daß das Biopolymer ein Polypeptid mit orts-, struktur und gewebespezifischen Eigenschaften ist.

6. Mikrokapseln gemäß Anspruch 1-5 zur Anwendung in der Ultraschall-Diagnostik, dadurch gekennzeichnet, daß das Wandmaterial ein Gas, bevorzugt Luft, Stickstoff, Kohlendioxid, Sauerstoff, Helium, Neon, Argon, Krypton oder ein Gemisch aus mindestens zwei dieser Gasen, einschließt.

7. Mikrokapseln gemäß Anspruch 1-5, dadurch gekennzeichnet, daß der über die funktionellen Gruppen des Biopolymeren gebundenen Reste chelatierende Liganden sind.

8. Mikrokapseln gemäß Anspruch 1-5 und 7 enthaltend als chelatisierende Liganden Ethylendiaminpentaessigsäure-Reste oder deren Derivate.

9. Mikrokapseln gemäß Anspruch 1-5, 7 und 8, dadurch gekennzeichnet, daß die über die funktionellen Gruppen des Biopolymeren gebundenen Reste Chelat-Komplexe eines Metallions sind.

10. Mikrokapseln gemäß Anspruch 1-5 und 7-9, dadurch gekennzeichnet, daß die komplex gebundenen Metallionen paramagnetisch, bevorzugt Gadoliniumionen sind.

11. Mikrokapseln gemäß Anspruch 9-10 zur Anwendung in der NMR-Diagnostik.

12. Mikrokapseln gemäß Anspruch 1-5 und 7-9, dadurch gekennzeichnet, daß die komplex gebundenen Metallionen Radiolsotope, bevorzugt ^{99m}Technetium- oder ¹¹¹Indium-Ionen sind.

13. Mikrokapseln gemäß Anspruch 12 zu Anwendung in der Szintigraphie.

14. Verfahren zur Herstellung von Mikrokapseln gemäß Anspruch 1, dadurch gekennzeichnet, daß das Monomere in einer Konzentration von 0,01 - 10 % (m/V) , bevorzugt 0,1 - 10 % bezogen auf das Gesamtvolumen der Herstellungslösung unter Dispergieren in gasgesättigter, gegebenenfalls autoklavierter, wässriger Phase, die ein gelöstes Biopolymer aus der Gruppe der Polypeptide oder der Gruppe der Collagenabbauprodukte in einer Konzentration von 0,5 - 20 % (m/V) bevorzugt 1 % - 15 % (m/V)), sowie gegebenenfalls zusätzlich magnetische Partikel enthält, polymerisiert wird, unter der Maßgabe, daß das Gewichtsverhältnis von Biopolymerem zu synthetischem Polymeren 10:90 bis 80:20 ist, nach beendeter Polymerisation die entstandenen Mikropartikel je nach Dichte und Partikelgröße durch einmalige oder mehrmalige Zentrifugation, Filtration, Sedimentation oder Flotation abtrennt, gegebenenfalls durch Dialyse weiter reinigt und in einem physiologisch verträglichen Suspensionsmittel suspendiert und anschließend gegebenenfalls mit Chelatbildnern, Metallchelaten und/oder orts-, struktur-oder gewebespezifischen Substanzen umsetzt und diese Suspension, bevorzugt unter Zumischung von Gerüstbildnern, gefriertrocknet.

15. Kontrastmittel, dadurch gekennzeichnet, daß die Mikropartikel nach Anspruch 1 in einem pharmazeutisch akzeptablen Suspensionsmedium resuspendiert werden.

16. Kontrastmittel gemäß Anspruch 15, dadurch gekennzeichnet, daß als pharmazeutisch akzeptables Suspensionsmedium Wasser für Injektionszwecke, das gegebenenfalls einen Zusatz von Kochsalz, Glucose, Mannitol und/oder Lactose und gegebenenfalls zusätzlich einen mehrwertigen Alkohol enthält, verwendet wird.

17. Kontrastmittel herstellbar nach dem in den Ansprüchen 14 beschriebenen Verfahren, dadurch gekennzeichnet, daß auf eine abschließende Gefriertrocknung verzichtet wird.

## Claims

1. Microcapsules for diagnosis and/or treatment comprising biodegradable polymers, characterised in that
the wall material comprises a mixed polymer of at least one synthetic polymeric material and at least one biopolymer from
(i) the group of polypeptides, which are optionally glycosylated, or
(ii) the group of collagen degradation products,
the biopolymer
a) having location-, structure- or tissue-specific properties or
b) having functional groups *via* which chelating ligands or metal complexes thereof and/or location-, structure- or tissue-specific substances are optionally bonded,
and the wall material optionally additionally contains one or more pharmaceutical active ingredient(s),
and the core of the capsules comprises
a) a gas or gas mixture and/or
b) a pharmaceutical active ingredient or active ingredient mixture or
c) the same material as the capsule wall,
with the provisos that the synthetic polymeric material is not formed from polymerisable aldehydes, that the ratio by weight of biopolymer to synthetic polymer is in the range of from 10:90 to 80:20, and that the size of the micro-capsules is from 0.5 to 8 µm.

2. Microcapsules according to claim 1, characterised in that the synthetic polymer is formed from monomeric acrylic acid, acrylamide, acrylic acid chloride, acrylic acid glycide ester or monomeric alkylcyanoacrylates.

3. Microcapsules according to claim 1, characterised in that the biopolymer is albumin, fibrinogen, fibronectin, gelatin, polygelin, oxypolygelatin or poly-L-lysine.

4. Microcapsules according to claim 1, characterised in that the location-, structure- and tissue-specific substances optionally bonded *via* the functional groups of the biopolymer are antibodies, conjugated antibodies, hormones, transferrin, fibronectin, heparin, transcobalamin, epidermal growth factor, lipoproteins, plasma proteins, peptides or oligopeptides, which preferably contain the amino acid sequences RGD, RGDS, RGDV or RGDT.

5. Microcapsules according to claim 1, characterised in that the biopolymer is a polypeptide having location-, structure- and tissue-specific properties.

6. Microcapsules according to claim 1 - 5 for use in ultrasound diagnostics, characterised in that the wall material encloses a gas, preferably air, nitrogen, carbon dioxide, oxygen, helium, neon, argon, krypton or a mixture of at least two of those gases.

7. Microcapsules according to claim 1 - 5, characterised in that the radicals bonded *via* the functional groups of the biopolymer are chelating ligands.

8. Microcapsules according to claim 1 - 5 and 7 containing as chelating ligands ethylenediaminepentaacetic acid radicals or derivatives thereof.

9. Microcapsules according to claim 1 - 5, 7 and 8, characterised in that the radicals bonded *via* the functional groups of the biopolymer are chelate complexes of a metal ion.

10. Microcapsules according to claim 1 - 5 and 7 - 9, characterised in that the complex-bound metal ions are paramagnetic preferably being gadolinium ions.

11. Microcapsules according to claim 9 - 10 for use in NMR diagnostics.

12. Microcapsules according to claim 1 - 5 and 7 - 9, characterised in that the complex-bound metal ions are radioisotopes, preferably being ^{99m}technetium or ¹¹¹indium ions.

13. Microcapsules according to claim 12 for use in scintigraphy.

14. Process for the preparation of microcapsules according to claim 1, characterised in that the monomer is polymerised in a concentration of from 0.01 to 10 % (m/v), preferably from 0.1 to 10 %, based on the total volume of the preparation solution, with dispersion in a gas-saturated, optionally autoclaved, aqueous phase that contains a dissolved biopolymer from the group of polypeptides or the group of collagen degradation products in a concentration of from 0.5 to 20 % (m/v), preferably from 1 to 15 % (m/v), and optionally additionally magnetic particles, with the proviso that the ratio by weight of biopolymer to synthetic polymer is from 10:90 to 80:20, when the polymerisation is complete the resulting microparticles are separated according to density and particle size by centrifugation, filtration, sedimentation or flotation carried out one or more times, optionally purified further by dialysis and suspended in a physiologically tolerable suspension agent and then optionally reacted with chelating agents, metal chelates and/or location-, structure-or tissue-specific substances, and the suspension is freeze-dried, preferably with the admixture of structure-formers.

15. Contrast medium, characterised in that the microparticles according to claim 1 are resuspended in a pharmaceutically acceptable suspension medium.

16. Contrast medium according to claim 15, characterised in that the pharmaceutically acceptable suspension medium used is water for injection that optionally contains an addition of sodium chloride, glucose, mannitol and/or lactose and optionally additionally a polyhydric alcohol.

17. Contrast medium preparable in accordance with the process described in claim 14, characterised in that final freeze-drying is omitted.

## Revendications

1. Microcapsules pour le diagnostic et/ou la thérapie en polymères biodégradables, caractérisées en ce que
la matière de paroi est constituée d'un copolymère d'au moins une matière polymère synthétique et d'au moins un biopolymère pris
(i) dans le groupe des polypeptides, qui sont éventuellement glycosylés, ou
(ii) dans le groupe des produits de dégradation du collagène, le biopolymère
a) présentant des caractéristiques spécifiques du site, de la structure ou du tissu, ou
b) possédant des groupes fonctionnels par l'intermédiaire desquels sont liés éventuellement des liants chélatants ou leurs complexes métalliques et/ou des substances spécifiques du site, de la structure ou du tissu,
et la matière de paroi comporte éventuellement en plus un ou plusieurs principes actifs pharmaceutiques,
et en ce que le moyau des capsules est constitué
a) d'un gaz ou d'un mélange de gaz, et/ou
b) d'un principe actif pharmaceutique ou d'un mélange de principes actifs pharmaceutiques, ou
c) de la même matière que la paroi des capsules,
à la condition que la matière polymère synthétique ne soit pas constituée en aldéhydes polymérisables, en ce que le rapport pondéral du biopolymère au polymère synthétique soit dans l'intervalle de 10:90 à 80:20, et que la taille des microcapsules soit de 0,5 à 8 µm.

2. Microcapsules selon la revendication 1, caractérisées en ce que le polymère synthétique est obtenu à partir d'acide acrylique, d'acrylamide, de chlorure d'acide acrylique, d'acrylates de glycidyle monomerès ou des cyanoacrylates d'alkyle monomères.

3. Microcapsules selon la revendication 1, caractérisées en ce que le biopolymère est l'albumine, le fibrinogène, la fibronectine, la gélatine, la polygéline, l'oxypolygélatine ou la poly-L-lysine.

4. Microcapsules selon la revendication 1, caractérisées en ce que les substances spécifiques du site, de la structure et du tissu, liés éventuellement par l'intermédiaire des groupes fonctionnels du biopolymère, sont des anticorps, des anticorps conjugués, des hormones, la transferrine, la fibronectine, l'héparine, la transcobalamine, le facteur de croissance épidermique, des lipoprotéines, des protéines plasmatiques, des peptides ou des oligopeptides, qui contiennent de préférence des séquences d'acides aminés RGD, RGDS, RGDV ou RGDT.

5. Microcapsules selon la revendication 1, caractérisées en ce que le biopolymère est un polypeptide ayant des caractéristiques spécifiques du site, de la structure et des tissus.

6. Microcapsules selon les revendications 1 à 5, pour utilisation en diagnostic par ultrasons, caractérisées en ce que la matière de paroi renferme un gaz, de préférence l'air, l'azote, le dioxyde de carbone, l'oxygène, l'hélium, le néon, l'argon, le krypton ou un mélange d'au moins deux de ces gaz.

7. Microcapsules selon la revendication 1 à 5, caractérisées en ce que les restes liés par l'intermédiaire des groupes fonctionnels du biopolymère sont des ligands.

8. Microcapsules selon la revendication 1 à 5 et 7, contenant en tant que ligand chélatant des restes d'acide éthylènediaminopentaacétique ou ses dérivés.

9. Microcapsules selon la revendication 1 à 5, 7 et 8, caractérisées en ce que les restes liés par l'intermédiaire des groupes fonctionnels du biopolymère sont des complexes chélatés d'un ion métallique.

10. Microcapsules selon la revendication 1 à 5 et 7 à 9, caractérisées en ce que les ions liés de façon complexe sont paramagnétiques, de préférence des ions gadolinium.

11. Microcapsules selon la revendication 9 à 10 pour des applications dans le diagnostic par la RMN.

12. Microcapsules selon la revendication 1 à 5 et 7 à 9, caractérisées en ce que les ions métalliques liés de façon complexe sont des radioisotopes, de préférence ^{99m}Technetium ou ¹¹¹Indium.

13. Microcapsules selon la revendication 12 pour des applications en scintigraphie.

14. Procédé de préparation de microcapsules selon la revendication 1, caractérisé en ce que l'on polymérise le monomère dans une concentration de 0,01 à 10 % (m/V), de préférence de 0,1 à 10 % par rapport au volume total de la solution de préparation, en dispersant dans une phase aqueuse, éventuellement autoclavée, saturée en gaz, qui contient un biopolymère dissous pris dans le groupe des polypeptides ou du groupe des produits de dégradation du collagène dans une concentration de 0,5 à 20 % (m/V), de préférence de 1 % à 15 % (m/V), ainsi qu'éventuellement en plus des particules magnétiques, à la condition que le rapport pondéral du biopolymère au polymère synthétique soit de 10:90 à 80:20, après avoir terminé la polymérisation, on sépare les microparticules formées selon la densité et la taille des particules par une ou plusieurs centrifugations, filtrations, sédimentations ou flottations, éventuellement on les purifie davantage par dialyse et on les met en suspension dans un agent de suspension physiologiquement compatible, et ensuite on les fait réagir éventuellement avec des agents chélatants, des chélates métalliques, et/ou des substances spécifiques du site, de la structure ou des tissus, et on lyophilise cette suspension de préférence en ajoutant des agents formateurs de charpente.

15. Produit de contraste, caractérisé en ce que les microparticules selon la revendication 1 sont resuspendues dans un milieu de suspension pharmaceutiquement acceptable.

16. Produit de contraste selon la revendication 15, caractérisé en ce l'on utilise en tant que milieu de suspension pharmaceutiquement acceptable l'eau pour injection, qui contient éventuellement un ajout de chlorure de sodium, de glucose, de mannitol et/ou de lactose, et éventuellement en plus un alcool polyhydroxylique.

17. Produit de contraste que l'on peut préparer selon le procédé décrit dans la revendication 14, caractérisé en ce que l'on renonce à la lyophilisation ultérieure.
